Europäisches Patentamt

European Patent Office

Office européen des brevets

(19)

(11) Publication number : **0 646 316 A1**

(12) **EUROPEAN PATENT APPLICATION**

(21) Application number : **94306951.8**

(22) Date of filing : **22.09.94**

(51) Int. Cl.[6] : **A01N 63/04, C12P 19/04**

(30) Priority : **22.09.93 IL 10707593**
**25.07.94 IL 11044194**

(43) Date of publication of application :
**05.04.95 Bulletin 95/14**

(84) Designated Contracting States :
**AT BE CH DE DK ES FR GB GR IE IT LI NL PT SE**

(71) Applicant : **STATE OF ISRAEL-MINISTRY OF AGRICULTURE**
**Volcani Research Center**
**P.O. Box 6**
**IL-50 250 Bet Dagan (IL)**

(72) Inventor : **Droby Samir**
**10/12 Baka-El-Garbea**
**30100 Israel (IL)**
Inventor : **Chalutz Edo**
**13 Haem Street**
**Rishon Lezion75240 (IL)**
Inventor : **Wilson Charles**
**2004 Thanes Drive, Martinsburg**
**West Virginia 25401 (US)**
Inventor : **Wisniewski Michael**
**Rt. 1, Box 136 Shepherdstown**
**West Virginia 25443 (US)**

(74) Representative : **Hillier, Peter et al**
**Reginald W. Barker & Co.,**
**Chancery House,**
**53-64, Chancery Lane**
**London, WC2A 1QU (GB)**

(54) Fungicides and method for using same.

(57) Fungicides for treating fruit and vegetables comprising extracellular polymers (EP) isolated from fungi.

EP 0 646 316 A1

## FIELD AND BACKGROUND OF THE INVENTION

The present invention relates to novel fungicides, including fungicidal compositions, and a method for controlling diseases of fruit and vegetables. More specifically, the invention relates to fungicidal extracellular polymers derived from fungi.

Postharvest diseases of fruit and vegetables cause 15 to 25% losses yearly in the fruit and vegetable industry worldwide. The major weapon in combatting and controlling these diseases is chemical fungicides. These are sometimes inadequate and are often hazardous to humans and to the environment. Therefore, there is a constant incentive and need to find effective alternative products to combat these diseases, preferably products derived from nature.

It is known that by treating fruit and vegetables post-harvest with certain antagonistic microorganisms it is possible to control postharvest diseases. Among such antagonists, yeasts were reported to control postharvest diseases of fruit and vegetables, see Wilson and Wisniewski, Annu. Rev. Phytopathol. 27: 425-444 (1989), Droby et al., Postharvest News and Information 2: 169-173 (1991) and Wilson et al. U.S. Patent No. 5, 041,384. Another naturally derived fungicidal material is chitosan, a high molecular weight polysaccharide obtained from crab shell, which was reported to reduce decay in strawberries, see El Ghaouth et al., Phytopathol. 82: 389-402 (1992),

## SUMMARY OF THE INVENTION

It is an object of this invention to provide novel fungicides derived from natural sources. Another object of the invention is to provide fungicidal compositions for preventing and treating diseases in fruit and vegetables. It is a further object of this invention to provide fungicides derived from extracts of yeast and other fungi and fungicidal compositions containing them. Yet another object of this invention is to provide a method of preventing diseases in fruit and vegetables. Still another object of the invention is to provide a method of curing diseases in fruit and vegetables. A further object of the invention is to provide a method of preventing and treating postharvest diseases in fruit and vegetables.

Another object of the invention is to provide a process for preparing fungicidal compositions. Other objects of the invention will appear from the description which follows.

In accordance with this invention, there are thus provided novel fungicides for treating fruit and vegetables comprising extracellular polymers (EP) isolated from fungi. Thus, the fungicides of the invention may either consist essentially of the EPs or may comprise the EPs.

Yeasts, as mentioned above, were reported to possess antifungal properties. We have surprisingly discovered that the extracellular polymers (EP), extracted from fungi such as yeast cells, possess fungicidal activity similar to that of live yeast cells containing an equivalent concentration of unseparated cell-wall-bound polymers. However, the extracted extracellular polymers (EP) have the advantage that they retain their antifungal activity at all times, whereas whole yeast cells show antifungal activity only when they are alive.

While the exact composition of the extracellular polymers is not yet completely known, they appear to be comprised substantially of polysaccharides.

Compositions containing these extracellular polymers (EP) derived from fungi, have excellent fungicidal activity either when used as sole active ingredient or when used in combination with other fungicides, for example, chemical fungicides such as TBZ.

Illustrative examples of fungi from which extracellular fungicidal polymers can be derived are the strains tabulated in the following table:

2

| Strain | Origin | Location |
|--------|--------|----------|
| Pichia guilliermondii "US-7" | surface of lemons | NRRL* Y-18314 |
| Debaryomyces hansenii "114" | surfaces of oranges | Volcani♣ |
| Saccharomyces cerevisiae◆"CSC" | Baker's yeast | Sigma Chemical |
| Candida oleophila "182" | grapefruit surface | NRRL* Y-18844 |
| Aureobasidium pullulans | grapes | Volcani♣ |
| Saccharomyces bayanos EC1118 | wine | Lalvin♥ |
| Rhodotorula mucilaginosa | grapes | Volcani♣ |
| Colletotrichum gleoosporioides, | decayed avocados | Volcani♣ |
| Botrytis cinerea | decayed tomatoes | Volcani♣ |
| Penicillium digitatum | decayed grapefruit | Volcani♣ |

*Northern Regional Research Center, US Department of Agriculture, Peoria, Illinois 61604, USA
♣State of Israel - Ministry of Agriculture, The Volcani Center, P.O. Box 6, Bet Dagan 50250, Israel
◆Type II                    ♥Montreal HIW 2N8, Quebec, Canada

BRIEF DESCRIPTION OF THE DRAWINGS

The invention will be better understood with reference to the drawings, in which:
Figure 1 is a block graph showing the fungicidal effect of various concentrations of extracellular polymers (EP) derived from different yeasts;
Figure 2 illustrates in block graph form the effect of different extraction methods on fungicidal activity;
Figure 3 is a block graph showing the effect of nutrient on the fungicidal activity of 1) extracellular polymers (EP) extracted from yeast cells (US-7) and 2) unextracted whole live yeast cells (US-7);
Figure 4 is a block diagram showing the effect of the presence of extracellular polymers extracted from US-7 cells, on the fungicidal activity of whole 182 yeast cells; and
Figure 5 is a block diagram illustrating the effect of EP extracted from 182 cells on the natural decay of Shamouti oranges.

DETAILED DESCRIPTION OF THE INVENTION

In the fungicides according to the invention, the EPs may be prepared by a method comprising subjecting cells of fungi to at least steps (i) and (ii) of the following sequential steps (i), (ii) and (iii): (i) sonication, or vigorous agitation with aqueous LiCl solution; (ii) removal of the cells and, where used, the LiCl; (iii) chromatographic purification. Persons skilled in the art will appreciate that use of an aqueous LiCl solution in the manner indicated is merely exemplary of the method which may be used to obtain the extracellular polymers of the invention, and that chemically equivalent methods of extraction may be used. Preliminary experiments by the inventors have shown that, e.g., the following reagents in aqueous solution may be used in place of aqueous LiCl in order to obtain the extracellular polymers: dithiothreitol + Tris-HCl buffer (pH 7.4), or 0 .1N NaOH, or sodium dodecyl sulfate/mercaptoethanol/urea in phosphate buffer (pH 6.5), or NaCl + EDTA.

Fungicides according to the present invention may be further characterized in that they comprise additionally at least one of (a), (b) and (c): (a) whole yeast cells; (b) at least one inert carrier, diluent or adjuvant;

3

(c) known fungicides other than known fungicidal yeast cells.

**Extraction of Extracellular Polymers (EP) from Yeast using LiCl**

Extracellular polymers were extracted from yeast cultures grown in 500 ml Erlenmeyer flasks or in a 10 liter fermenter. The growth medium used was NYDB medium containing: nutrient broth, 8 g; yeast extract, 5 g; and glucose, 10 g. At stationary phase (48 h incubation at 24°C on a rotary shaker at 120 rpm or fermentation) cells were pelleted by centrifugation at 7000 rpm for 20 min. Fresh weight of cell yield was approximately 20 g per liter of yeast culture.

The supernatant (culture medium) was discarded and the pelleted yeast cells were suspended in a salt solution containing 5 M LiCl in 10 mM Tris/HCl buffer, pH 7.4 (5 g of yeast cells per 50 ml of salt solution). The cells were agitated vigorously in a horizontal shaker for 1 hr. at room temperature. The cell suspension was then centrifuged at 7000 rpm for 20 min and the supernatant was collected and passed through a Millipore filter (0.45µm pore size) to remove any remaining cells. The volume of the extract was reduced to about one fifth by Rotavapor at 50 °C. In order to remove the LiCl, the concentrated extract was dialyzed using dialysis tubing (3 cm in diameter, 1000 MW cut-off) against 3 changes of distilled water, followed by an overnight dialysis under the same conditions. The dialysate was then freeze-dried and stored as a powder at -18°C until use.

The active components were further purified using gel filtration chromatography on columns containing TSK gel. Biological activity of the active fraction was increased 5-6 fold as compared with the non-purified extract. The active fraction detected following gel filtration was subjected to additional purification by HPLC to obtain highly purified material for identification purposes.

**Extraction of Extracellular Polymers (EP) from Yeast using sonication**

Yeast cultures were grown in 500 ml Erlenmeyer flasks containing NYDB medium. At stationary phase, after 48 hours incubation at 24°C on a rotary shaker at 120 rpm, the cells were pelleted by centrifugation at 7000 rpm for 20 minutes. Fresh weight of cell yield was approximately 20 g per liter of yeast culture. The supernatant culture medium was discarded and the pelleted cells were suspended in phosphate buffer (pH 7) and subjected to sonication for 30 seconds using a sonicator (Ultrasonics 225) at 30 % output. Following sonication, cells were pelleted as already described and the supernatant was filtered through a Millipore filter (0.45µm pore size) to remove any remaining cells, and the volume of the extract was reduced to about one fifth by Rotavapor at 50 °C. The concentrated extract was freeze-dried and stored as a powder at -18°C until required for use.

**Extraction of Extracellular Polymers (EP) from <u>Colletotrichum gleoosporioides</u>, <u>Botrytiscinereaand Penicilliumdigitatum</u>using LiCl**

Fungi were grown in potato dextrose agar (PDB) for one week in a rotary shaker at 20 °C. Fungal mycelium was separated from the growth medium by filtration through filter paper and then shaken vigorously for one hour with 5mM LiCl in 10 mM Tris/HCl buffer (ph 7.4) as described above. The rest of the extraction procedure is as described for yeasts.

**Bioassay of Antifungal Activity of EP in Culture**

The effect of the EP extracted from the various fungal and yeast isolates on spore germination and germ tube elongation of postharvest pathogens was tested in a minimal salts medium. The medium contained : glucose, 10 g; L - asparagine, 2 g; $KH_2PO_4$, 1 g; $MgSO_4 \cdot 7H_2O$, 0.5g; $FeSO_4 \cdot 7H_2O$, 0.01 mg;

$ZnSO_4 \cdot 7H_2O$, 8.7 mg. Spore suspension of the various fungi tested, at a concentration of $10^5$ spores/ml were prepared in the minimal salts medium. The EP preparation was dissolved in sterile distilled water and added to 1 ml aliquots of the spore suspension to reach the desired final concentration. Then, 30 μl drops containing fungal spores and EP were placed on ethanol-washed microscope slides (3 drops per slide) and incubated on moist filter paper in Petri plates. Spore germination and germ tube elongation were observed under a light microscope following 18 hrs. incubation at 22°C. Percent germination and germ tube length in each replicate was determined in three microscopic fields, each containing 200 - 300 spores.

The following plant pathogens were found to be affected in vitro by the EP preparations: Penicillium digitatum; Penicillium expansum; Penicillium italicum; Botrytis cinerea; Monilinia fructicula; and Rhizopus stolonifer.

The EP preparation was found effective in inhibiting the infection and development of: 1. postharvest decay of citrus; 2. postharvest decay of apple; 3. postharvest decay of cut surface in banana hands; and 4. postharvest decay of cut surface in corn.

The fungicidal compositions may contain from 0.01% to about 1.0% extracellular polymers (EP).

In laboratory experiments, concentrations of 10 -1000 microliters/ml active ingredient were effective in treating infected fruit and vegetables and in preventing infection by fruit-rot pathogens.

The fungicidal compositions can be applied in any conventional manner, such as spraying and dipping. The exact amount of fungicide to be applied to obtain best results will have to be determined empirically.

**Experiments**

(1) The fungicidal activity of the extracellular polymer (EP), extracted as described above from US-7, on spore germination and germ tube elongation on various phytopathogenic fungi, is shown in Table 1. All of the pathogens listed are fungi that cause diseases of fruit and vegetables before and after harvest.

Table 1. FUNGICIDAL ACTIVITY OF EP FROM US-7 ON PHYTOPATHOGENIC FUNGI

| Pathogen | Spore germination | | Germ tube elongation |
|---|---|---|---|
| | $EC_{50}$ μg/ml | 100% Inhibition μg/ml | $EC_{50}$ μg/ml |
| Penicillium digitatum | 193 | 1000 | 196 |
| P. italicum | 239 | 1000 | 206 |
| P. expansum | NE | >2000 | 798 |
| Botrytis cinerea | 662 | 1000 | 552 |
| Rhizopus stolonifer | 1031 | 2000 | 807 |
| Monilinia fructicula | 194 | 500 | 97 |

NE= No effect on spore germination
$EC_{50}$=spore germination and germ tube elongation calculated by regression equations of Probit of percentage of inhibition.

The results listed in Table 1 show that Penicillium digitatum, P. italicum and Monilinia fructicula were the most sensitive to the presence of the extracellular polymers.

(2) The fungicidal effect of extracellular polymers (EP) extracted as described above from various yeast isolates on spore germination and germ tube elongation of Penicillium digitatum, which causes green mold decay in citrus, is shown in Table 2.

The data presented in Table 2 demonstrate that extracellular polymers exhibiting fungicidal activity could be extracted from various yeasts.

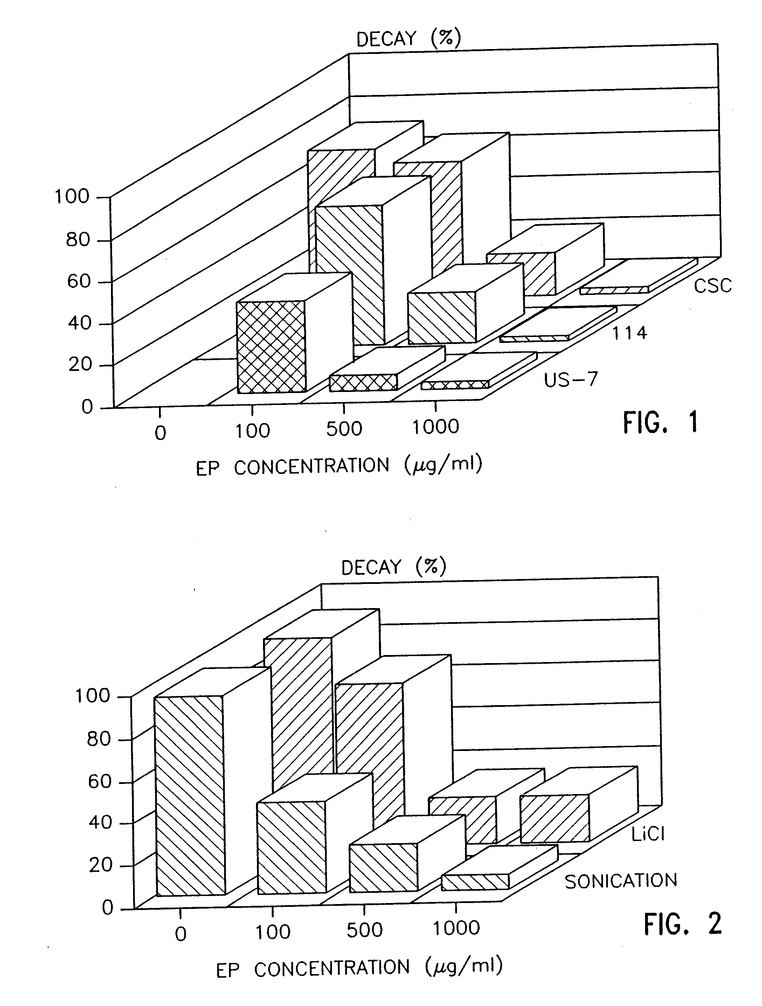

Figure 1 shows the effect of various concentrations of extracellular polymers extracted from different yeasts on wounded grapefruit; the data show that at concentrations of 500 and 1000 µg/ml, the extracted material effectively inhibited the development of green mold decay.

Table 2. FUNGICIDAL ACTIVITY OF EP FROM DIFFERENT YEASTS

| Yeast isolate | Spore germination $EC_{50}$ (µg/ml) | Germ tube elongation $EC_{50}$ (µg/ml) |
|---|---|---|
| US-7 | 106.8 | 74 |
| 114 | 151 | 228 |
| CSC | 62.3 | 63 |
| 182 | 105 | 86.1 |
| A. pullulans | 137 | 127 |
| S. bayanos | 106.8 | 98.8 |
| R. mucilaginosa | 186.8 | 174.2 |

Figure 2 shows the effect of extracellular polymers extracted from US-7 by different methods, on the development of green mold decay on wounded grapefruit. The results show that whereas the fungicidal activity of the extracellular polymers was not affected significantly by the extraction procedure when tested at a concentration of 500 µg/ml, extraction by sonication was preferable in relation to the tests at 100 and 1000 µg/ml.

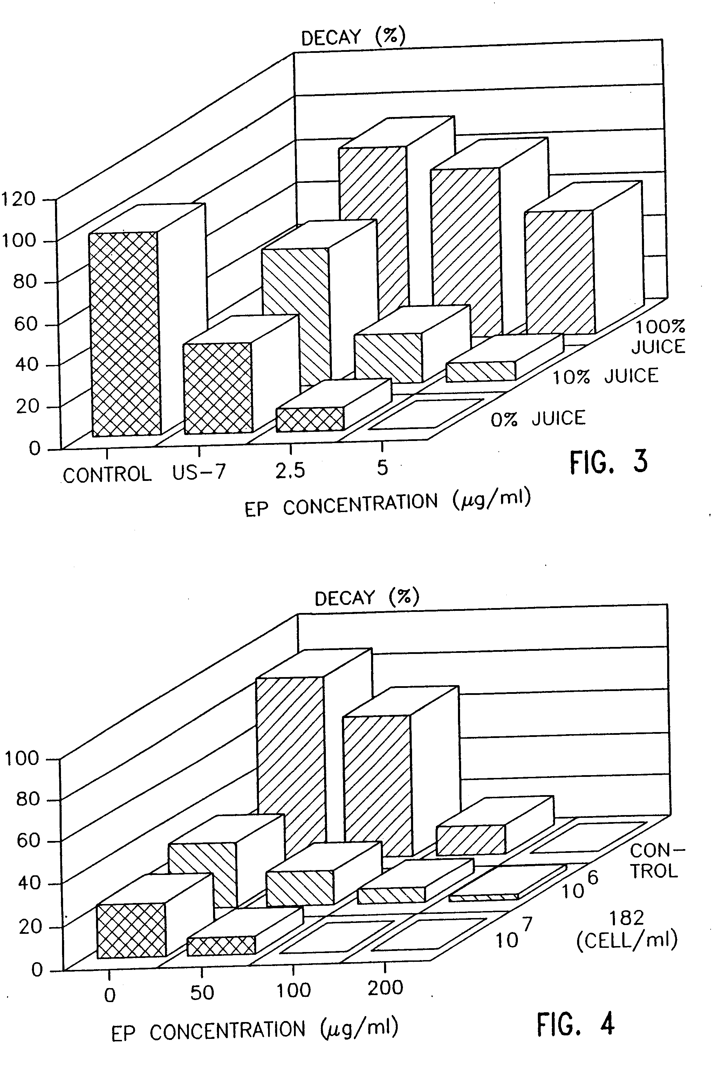

Figure 3 shows the effect of fruit juice on the fungicidal activity of extracellular polymers extracted from US-7, on the development of green mold decay on wounded grapefruit, and compares this activity with that of live US-7 cells. The results show that the fungicidal activity of the extracellular polymers could be reversed by increasing the concentration of grapefruit juice added to the extracellular polymer solution.

(3) A comparison of the fungicidal activity of extracted extracellular polymers (EP) from the yeast Pichia guilliermondii (US-7) vs. live unextracted yeast cells, against the pathogen Penicillium digitatum in infected grapefruit wounds, is shown in Table 3. The percent of infected wounds were determined after one week incubation at 25°C. In all cases the extracted fungicide in aqueous solution was applied to the areas infected or prone to infection in concentrations as indicated.

The results show that the extracellular polymers (EP) exhibited a very high inhibitory activity against the development of green mold decay on wounded grapefruit at concentrations of 500 and 1000 µg/ml; this activity was of the same order as that of live yeast cells at a concentration of $10^9$ cells/ml.

Table 3. COMPARISON OF FUNGICIDAL ACTIVITY OF EP VS. WHOLE YEAST

| Treatment | Infection % |
|---|---|
| EP conc.(µg/ml) | |
| 0 | 70.5 |
| 100 | 51.5 |
| 500 | 7.4 |
| 1000 | 3.8 |
| Pichia guilliermondii (cells/ml) | |
| 0 | 85.0 |
| $10^7$ | 39.7 |
| $10^8$ | 19.8 |
| $10^9$ | 3.3 |

Figure 4 shows the inhibition activity of EP (extracted from the US-7 isolate of Pichia guilliermondii), alone and together with whole yeast cells of the 182 yeast antagonist, with respect to the retardation of green mold decay of wounded grapefruit, artificially inoculated with the pathogen.

As can be seen in the figure, a concentration of $10^0$ or $2^{00}$ ug/ml of the EP alone, considerably reduced percent infection. In combination with relatively low concentrations of cell suspension of the antagonist, the effect was further enhanced.

(4) Table 4 shows the effect of EP (extracted from the antagonist 182) on preventing natural infection of wounded grapefruit, momentarily dipped in a solution of EP at two concentrations, and subsequently stored for 7-11 days at $2^0$ °C. The tests were carried out on a relatively large scale (3 cartons of fruit each containing 35 fruits, 3 wounds/fruit).

Table 4. EFFECT OF EP(182) ON NATURAL INFECTION OF WOUNDED GRAPEFRUIT (% DECAY).

| Incubation time (days) | Control | EP (300 µg/ml) | EP (1000 µg/ml) |
|---|---|---|---|
| 7 | 57.9 | 0.9 | 0.7 |
| 9 | 67.6 | 3.4 | 2.5 |
| 11 | 73.6 | 5.7 | 5.5 |

The results show that both concentrations of EP ($3^{00}$ and $1^{000}$ µg/ml) used in this experiment were very effective in controlling the development of natural decay on wounded grapefruit, com-

7

pared with untreated control fruits.

Table 5 illustrates results of an experiment on a similar scale (3 cartons of fruit each containing 32 fruits, 3 wounds/fruit) to that of Table 4, but lower concentrations of EP were used, and the effect was compared with two concentrations (in cells/ml) of yeast cell suspensions. An additional treatment tested the effect of a combination of yeast cells with EP.

Table 5. EFFECT OF EP(182) ON NATURAL INFECTION OF WOUNDED GRAPEFRUIT.

| Days* | Control | EP 100♦ | EP 200♦ | EP 500♦ | 182 $10^7$♥ | 182 $10^8$♥ | EP(100♦)+ 182($10^7$♥) |
|---|---|---|---|---|---|---|---|
| 5 | 27.4±3.82 | 1.1±0.8 | 0 | 0 | 12.7±2.46 | 11.0±2.24 | 3.06±1.42 |
| 7 | 43.6±1.7 | 5.8±0.94 | 1.9±0.92 | 0 | 29.0±2.0 | 17.8±3.0 | 8.6 ±0.27 |
| 12 | 81.2±1.23 | 10.0±1.54 | 4.5±0.33 | 2.3±0.65 | 66.3±2.3 | 43.7±7.0 | 20.0 ±0.41 |

*incubation time ♦µg/ml ♥cells/ml

The results show that EP at a concentration as low as $10^0$ µg/ml could effectively inhibit the development of natural decay on wounded grapefruit when used alone in aqueous solution. In addition, combination of EP with live yeast (182) cells at a low concentration ($10^7$ cells/ml) dramatically enhanced its biocontrol activity against the development of green mold decay on grapefruit.

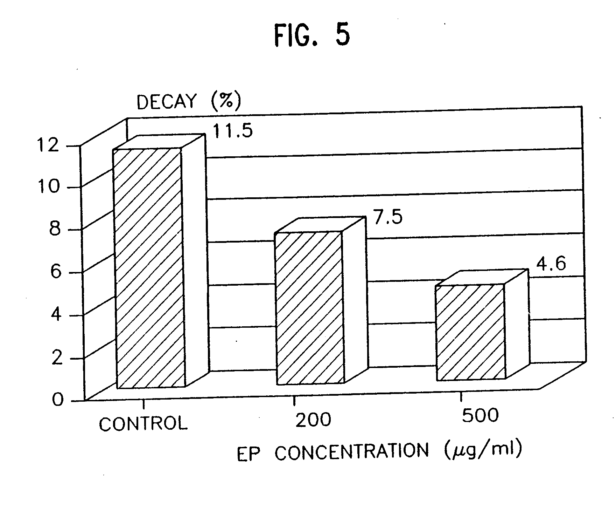

Figure 5 illustrates the results of a large scale pilot test in which two concentrations ($2 \times 10^0$ and $5 \times 10^0$ µg/ml) of EP (extracted from the antagonist 182) were applied to whole Shamouti oranges which were subsequently incubated for 9 days at $2 \times 10^0$ °C. Each treatment comprised $2 \times 10^0$ cartons of fruit, each carton containing 35-$4 \times 10^0$ fruits. The results show that concentrations of $2 \times 10^0$ and $5 \times 10^0$ µg/ml EP reduced the development of decay in the oranges, from 11.5% (control), to 7.5 and 4.6%, respectively.

(5) Extracellular Polymers (EP), extracted from Colletotrichum gleoosporioides, Botrytis cinerea and Penicillium digitatum, as described above, were tested for fungicidal activity against green mold decay in grapefruit. Results are given in Table 6.

Table 6. EFFECT OF EP FROM (182) ON 3 FUNGI ON ARTIFICIAL INFECTION OF WOUNDED GRAPEFRUIT (INCUBATION TIME = 5 DAYS).

| EP (µg/ml) | Infection (% of control) | | |
|---|---|---|---|
| | Colletotrichum gleoosporioides | Botrytis cinerea | Penicillium digitatum |
| 100 | 70 | 100 | 100 |
| 300 | 67.4 | 61.6 | 36 |
| 500 | 29.6 | 34.8 | 15.6 |
| 1000 | 0 | 9.3 | 6.0 |

The results show that decay was reduced by about 70-85% when using EP at a concentration of 500 μg/ml and by about 90-1000% when using EP at a concentration of 1000 μg/ml.

While the present invention has been particularly described with respect to its presently preferred embodiments, it will be appreciated by skilled persons that many modifications and variations may be made. Merely by way of example, chemically equivalent methods of extracting the extracellular polymers may be used in place of the methods specifically described herein. Consequently, it will be evident that the invention is not to be construed as restricted to the particularly described embodiments, rather regard will be had to the concept, spirit and scope of the invention, in view of the present disclosure and the claims which follow.

## Claims

1. Fungicides for treating fruit and vegetables comprising extracellular polymers (EP) isolated from fungi.

2. Fungicides according to claim 1 wherein the extracellular polymers (EP) are isolated from yeasts.

3. Fungicides according to claim 1 wherein the extracellular polymers (EP) are isolated from at least one member of the group consisting of Pichia, Debaryomyces, Saccharomyces, Candida, Aureobasidium, Rhodotorula, Colletotrichum, Botrytis and Penicillium.

4. Fungicides according to claim 4 wherein the extracellular polymers (EP) are isolated from at least one member of the group consisting of Pichia guilliermondii, Debaryomyces hansenii, Saccharomyces cerevisiae, Candida oleophila, Aureobasidium pullulans, Saccharomyces bayanos, Rhodotorula mucilaginosa, Colletotrichum gleoosporioides, Botrytis cinerea and Penicillium digitatum.

5. Fungicides according to any of the preceding claims, wherein the extracellular polymers (EP) are comprised substantially of polysaccharides.

6. Fungicides according to any of the preceding claims and which are effective against plant pathogens selected from the group consisting of Penicillium digitatum, Penicillium expansum, Penicillium italicum, Botrytis cinerea, Monilinia fructicula and Rhizopus stolonifer.

7. Fungicides according to any of the preceding claims, wherein the extracellular polymers have been prepared by a method comprising subjecting cells of fungi to at least steps (i) and (ii) of the following sequential steps (i), (ii) and (iii):
(i) sonication, or vigorous agitation with aqueous LiCl solution;
(ii) removal of the cells and, where used, the LiCl;
(iii) chromatographic purification.

8. Fungicides according to any of the preceding claims, which are further characterized in that they comprise additionally at least one of (a), (b) and (c):
(a) whole yeast cells;
(b) at least one inert carrier, diluent or adjuvant;
(c) known fungicides other than known fungicidal yeast cells.

9. Fungicides according to any of the preceding claims, comprising 0.01-1.0% extracellular polymers (EP).

10. A method of preventing or treating fungal infection of fruit or vegetables, which comprises applying to the surface of the fruit or vegetables a fungicide as defined in any of the preceding claims.

FIG. 1

FIG. 2

FIG. 3

FIG. 4

## FIG. 5

European Patent Office

**EUROPEAN SEARCH REPORT**

Application Number

EP 94 30 6951

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DATABASE WPI Week 9044, Derwent Publications Ltd., London, GB; AN 90-332321 [44] & JP-A-2 240 007 (HIGETA SHOYU) 25 September 1990 * abstract * | 1,5-9 | A01N63/04 C12P19/04 |
| Y | | 2-4,10 | |
| X | DATABASE WPI Week 9117, Derwent Publications Ltd., London, GB; AN 91-122560 [17] & JP-A-3 063 203 (HIGETA SHOYU) 19 March 1991 * abstract * | 1,5,8,9 | |
| Y | | 2-4,6,10 | |
| Y | WO-A-91 01641 (THE UNITED STATES OF AMERICA) * page 1, line 10 - page 4, line 3 * * page 6, line 18 - page 8, line 13 * * page 15, line 5 - page 18, line 23 * | 2-4,6,10 | TECHNICAL FIELDS SEARCHED (Int.Cl.6) A01N |
| Y | WO-A-92 18009 (DARATECH) * page 1, line 5 - line 9 * * page 3, line 11 - line 16 * | 2-4,6,10 | |
| Y | DATABASE WPI Week 8845, Derwent Publications Ltd., London, GB; AN 88-322575 [45] & US-A-7 177 236 (US SEC OF AGRICULTURE) * abstract * | 3,4,10 | |

-/--

The present search report has been drawn up for all claims

| Place of search | Date of completion of the search | Examiner |
|---|---|---|
| THE HAGUE | 11 November 1994 | Lamers, W |

EPO FORM 1503 03.82 (P04C01)

European Patent Office

# EUROPEAN SEARCH REPORT

Application Number

EP 94 30 6951

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | DATABASE WPI Week 8837, Derwent Publications Ltd., London, GB; AN 88-261551 & JP-A-63 190 808 (KOHRAN SANGYO) 8 August 1988 * abstract * --- | 3,4,6 | |
| X | DATABASE WPI Week 7821, Derwent Publications Ltd., London, GB; AN 78-37643A [21] & JP-A-53 041 423 (KANEGAFUCHI) 14 April 1978 * abstract * --- | 1-6 | |
| X | DATABASE WPI Week 7804, Derwent Publications Ltd., London, GB; AN 78-07120A [04] & JP-A-52 146 721 (KANEGAFUCHI) 6 December 1977 * abstract * --- | 2-6 | TECHNICAL FIELDS SEARCHED (Int.Cl.6) |
| X | DATABASE WPI Week 7430, Derwent Publications Ltd., London, GB; AN 74-54809V [30] & SU-A-402 539 (LENINGRAD RES INST) 16 March 1974 * abstract * --- -/-- | 1,3-6 | |

The present search report has been drawn up for all claims

| Place of search | Date of completion of the search | Examiner |
|---|---|---|
| THE HAGUE | 11 November 1994 | Lamers, W |

EPO FORM 1503 03.82 (P04C01)

14

European Patent Office

**EUROPEAN SEARCH REPORT**

Application Number

EP 94 30 6951

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DATABASE WPI<br>Week 7420,<br>Derwent Publications Ltd., London, GB;<br>AN 74-37669V [20]<br>& JP-A-49 016 634 (YAMASA SYOYU) 23 April 1974<br>* abstract * | 1,3-6 | |
| X | WO-A-89 12106 (INSTITUT PASTEUR)<br>* page 1, line 1 - line 4 *<br>* page 7, line 11 - line 23 * | 1,5,8,9 | |
| X | EP-A-0 396 750 (JAPAN TOBACCO)<br>* page 2, paragraph 3 *<br>* page 4, paragraph 4 * | 1,5,7-9 | |
| X | DATABASE WPI<br>Week 7901,<br>Derwent Publications Ltd., London, GB;<br>AN 79-01152B [01]<br>& JP-A-53 134 060 (KAKEN CHEM) 22 November 1978<br>* abstract * | 7 | TECHNICAL FIELDS SEARCHED (Int.Cl.6) |

The present search report has been drawn up for all claims

| Place of search | Date of completion of the search | Examiner |
|---|---|---|
| THE HAGUE | 11 November 1994 | Lamers, W |

EPO FORM 1503 03.82 (P04C01)